(19) Europäisches Patentamt
European Patent Office
Office européen des brevets

(11) Publication number: **0 059 966**
**B1**

(12) **EUROPEAN PATENT SPECIFICATION**

(45) Date of publication of patent specification: **13.08.86**

(51) Int. Cl.$^4$: **C 07 K 5/06,** A 61 K 37/02

(21) Application number: **82101797.7**

(22) Date of filing: **08.03.82**

(54) **Substituted thiazolidine carboxylic acid analogs and derivatives as antihypertensives.**

(30) Priority: **09.03.81 US 241957**
**21.01.82 US 340242**

(43) Date of publication of application:
**15.09.82 Bulletin 82/37**

(45) Publication of the grant of the patent:
**13.08.86 Bulletin 86/33**

(84) Designated Contracting States:
**AT BE CH DE FR GB IT LI LU NL SE**

(56) References cited:
**EP-A-0 012 401**
**EP-A-0 031 104**

(73) Proprietor: **MERCK & CO. INC.**
**126, East Lincoln Avenue P.O. Box 2000**
**Rahway New Jersey 07065 (US)**

(72) Inventor: **Patchett, Arthur A.**
**1090 Minisink Way**
**Westfield New Jersey 07090 (US)**
Inventor: **Wu, Mu Tsu**
**36 Lance Drive**
**Clark New Jersey 07066 (US)**

(74) Representative: **Abitz, Walter, Dr.-Ing. et al**
**Abitz, Morf, Gritschneder, Freiherr von**
**Wittgenstein Postfach 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 059 966 B1

**Description**

Background of the Invention

This invention, in its broad aspects, relates to substituted arylthiazolidine carboxylic acid analogs and derivatives which are potent angiotensin converting enzyme inhibitors useful in the treatment of hypertension.

Prior Art

EP—A 12 401 falling within the terms of Article 54, paragraph 3 refers to carboxyalkyl dipeptides wherein the carboxyl terminal amino acid may be proline inter alia. These compounds are useful as converting enzyme inhibitors.

Description of the Invention

The substituted arylthiazolidine carboxylic acid analogs and derivative compounds of this invention are represented by the following formula:

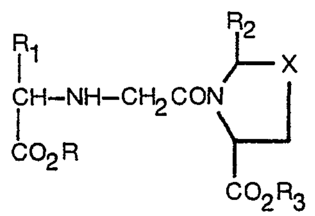

(I)

wherein;

R and $R_3$ are the same or different and are hydrogen, loweralkyl, aralkyl;

$R_{11}$ is alkyl of from one to ten carbon atoms which include, branched, cyclic and unsaturated alkyl groups; substituted alkyl of from one to six carbon atoms wherein the substituent is amino, arylthio, aryloxy, hydroxy, arylamino, and acylamino; aralkyl and heteroaralkyl substituted by halo, loweralkyl, hydroxy, aminoloweralkyl, alkoxy and amino groups and wherein the alkyl group contains from one to six carbon atoms;

$R_2$ is an aryl, substituted aryl, heteroaryl or substituted heteroaryl group selected from the group consisting of phenyl, naphthyl, or biphenyl wherein the substituent is hydroxyl or lower alkyloxy;

X is $CH_2$ or S; and,

the pharmaceutically acceptable salts thereof.

Preferred are those compounds of Formula I wherein:

R and $R_3$ are the same or different and are hydrogen, loweralkyl, aralkyl;

$R_1$ is alkyl of one to six carbon atoms optionally substituted by amino, hydroxyl, acylamino, aryloxy; aralkyl, and heteroaralkyl substituted by halo, aminoloweralkyl, hydroxy, alkoxy, and amino groups wherein the alkyl groups contain from one to six carbon atoms;

$R_2$ is phenyl, naphthyl, biphenyl, or 2-hydroxy-phenyl; and,

X is $CH_2$ or S.

More preferred are compounds of Formula I wherein:

R and $R_3$ are the same or different and are hydrogen or loweralkyl;

$R_1$ is aralkyl and heteroaralkyl both substituted by halo, hydroxy, and amino groups wherein the alkyl groups contain from one to four carbon atoms;

$R_2$ is 2-hydroxyphenyl or phenyl; and,

X is S.

The preferred and more preferred compounds of Formula I also include the pharmaceutically acceptable salts thereof.

The lower alkyl groups, except where noted otherwise, are straight or branched chain hydrocarbon radicals containing from one to six carbon atoms such as, for example, methyl, ethyl, isopentyl, and the like.

Halo means fluoro, bromo, chloro, or iodo.

In $R_1$, the aryl groups refer to phenyl, biphenyl, or naphthyl and the heteroaryl groups include, for example, pyridyl, thienyl, furyl, imidazolyl, quinolinyl, isoquinolinyl, indolyl, benzthenyl, thiazolyl, and the like.

Aralkyl groups refers to loweralkyl groups substituted by phenyl, biphenyl, or naphthyl.

Acyl refers to lower alkanoyl or aroyl.

The compounds of this invention are synthesized by the methods described below, wherein R, $R_1$, $R_2$, $R_3$ and X are as defined above, employing starting materials which are known to those skilled in the art and which are described in the literature. Reactive, interfering functionality in these starting materials such as, for example, hydroxy, phenolic, carboxylic acid, or amino groups, can be masked by protecting groups which can be subsequently removed to afford desired end products. Appropriate protecting groups are also known to those skilled in the art and described in the literature.

REACTION SCHEME

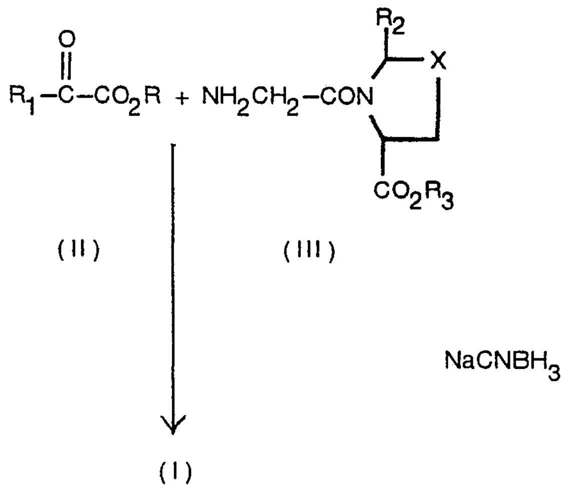


(II) (III)

(I)

Dipeptide III is prepared by coupling known, substituted imino carboxylic acids having the formula:

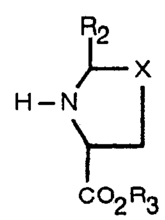

(IV)

with N-carbobenzyloxyglycine, N-*t*-butoxycarbonylglycine, N-phthaloylglycine, or similar N-protected glycine derivatives by activating the carboxyl group of these glycine derivatives as their acid chlorides and performing the coupling under basic, aqueous conditions or by using condensing agents such as, for example, carbodiimide or diphenyl phosphorylazide, followed by removal of the protecting groups by established methods known to those skilled in the art of peptide chemistry.

The condensation of compound III with compound II is conducted in an aqueous solution, optimally near neutrality, or in a suitable organic solvent; e.g., $CH_3CN$, in the presence of sodium cyanoborohydride to give compound I after removing the protecting groups, if any, by standard methods.

In products of Formula I, the carbon atoms to which $R_1$, $R_2$ and $CO_2R_3$ are attached are asymmetric so that diastereomers of Formula I are possible. Starting material IV can be used as a mixture of diastereomers or as a pure diastereomer. When mixtures of diastereomers are formed in the synthesis of compounds of Formula I, they may be separated, if desired, to afford the preferred diastereomer by chromatographic or fractional crystallization methods. In general, the absolute configuration preferred at the carbons to which $R_1$ and the $CO_2R_3$ groups are attached are those of an L-aminoacid configuration. Furthermore, $R_2$ is preferred in a *cis*-orientation relative to the $CO_2R_3$ group.

The compounds of this invention form salts with various inorganic and organic acids and bases which are also within the scope of the invention. Such salts include ammonium salts, alkali metal salts like sodium and potassium salts (which are preferred), alkaline earth metal salts like the calcium and magnesium salts, salts with organic bases e.g., dicyclohexylamine salts, N-methyl-D-glucamine, salts with amino acids like arginine, lysine and the like. Also salts with organic and inorganic acids may be prepared, e.g. HCl, HBr, $H_2SO_4$, $H_3PO_4$, methanesulfonic, toluenesulfonic, maleic, fumaric, camphorsulfonic. The non-toxic physiologically acceptable salts are preferred, although other salts are also useful, e.g., in isolating or purifying the product.

The salts may be formed by conventional means as by reacting the free acid or free base forms of the product with one or more equivalents of the appropriate base or acid in a solvent or medium in which the salt is insoluble, or in a solvent such as water which is then removed *in vacuo* or by freeze-drying or by exchanging the cations of an existing salt for another cation on a suitable ion exchange resin.

The compounds of this invention inhibit angiotensin converting enzyme and thus block conversion of the decapeptide angiotensin I to angiotensin II. Angiotensin II is a potent pressor substance. Thus, blood-pressure lowering can result from inhibition of its biosynthesis especially in animals and humans whose hypertension is angioternsin II related. Furthermore, converting enzyme degrades the vasodepressor

substance, bradykinin. Therefore, inhibitors of angiotensin converting enzyme may lower blood-pressure also by potentiation of bradykinin. Although the relative importance of these and other possible mechanisms remains to be established, inhibitors of angiotensin converting enzyme are effective antihypertensive agents in a variety of animal models and are useful clinically, for example, in many human patients with renovascular, malignant and essential hypertension. See, for example, D.W. Cushman et al., *Biochemistry 16,* 5484 (1977).

The evaluation of converting enzyme inhibitors is guided by *in vitro* enzyme inhibition assays. For example, a useful method is that of Y. Piquilloud, A. Reinharz and M. Roth, *Biochem. Biophys. Acta. 206,* 136 (1970) in which the hydrolysis of carbobenzyloxyphenylalanylhistidinylleucine is measured. *In vivo* evaluations may be made, for example, in normotensive rats challenged with angiotensin I by the technique of J. R. Weeks and *J. A. Jones, Proc. Soc. Exp. Biol. Med., 104,* 646 (1960) or in a high renin rat model such as that of S. Koletsky et al., *Proc. Soc. Exp. Biol. Med. 125,* 96 (1967).

Thus, the compounds of this invention are useful as antihypertensives in treating hypertensive mammals, including humans, and they can be utilized to achieve the reduction of blood pressure by formulating in compositions such as tablets, capsules or elixirs for oral administration or in sterile solutions or suspensions for parenteral administration. The compounds of this invention can be administered to patients in need of such treatment in a dosage range of 1 to 200 mg per patient generally given several times, thus giving a total daily dose of from 1 to 800 mg per day. The dose will vary depending on severity of disease, weight of patient and other factors which a person skilled in the art will recognize.

Also the compounds of this invention may be given in combination with other diuretics or antihypertensives. Typically, these are combinations whose individual per day dosages range from one-fifth of the minimally recommended clinical dosages to the maximum recommended levels for the entities when they are given singly. To illustrate these combinations, one of the antihypertensives of this invention, effective clinically in the range 5—200 milligrams per day, can be effectively combined at levels ranging from 1—200 milligrams per day with the following antihypertensives and diuretics in dose ranges per day as indicated:

hydrochlorothiazide (10—100 mg), timolol (5—60 mg), methyldopa (65—2000 mg), the pivaloyloxyethyl ester of methyldopa (30—1000 mg), indacrinone (25—150 mg), and 4-{3-{[2-(1-hydroxycyclohexyl)ethyl]-4-oxo-2-thiazolidinyl}propyl}benzoic acid (10—100 mg).

In addition, the triple drug combinations of hydrochlorothiazide (10—100 mg) plus amiloride (5—20 mg) plus converting enzyme inhibitor of this invention (1—200 mg) or hydrochlorothiazide (10—100 mg) plus timolol (5—60 mg) plus the converting enzyme inhibitor of this invention (1—200 mg) are effective combinations to control blood pressure in hypertensive patients.

The above dose ranges will be adjusted on a unit basis as necessary to permit divided daily dosage. Also, the dose will vary depending on the severity of the disease, weight of patient and other factors which a person skilled in the art will recognize.

Typically the combinations shown above are formulated into pharmaceutical compositions as discussed below.

About 1 to 100 mg of a compound or mixture of compounds of Formula I or a physiologically acceptable salt thereof is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharine; a flavoring agent such as peppermint, oil of wintergreen or cherry. When the dosage unit form is a capsule, it may contain, in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc. or a synthetic fatty vehicle like ethyl oleate or the like. Buffers, preservatives, antioxidants and the like can be incorporated as required.

The following example 6 is illustrative of the invention and constitutes especially preferred embodiments. The preferred diastereomers of these examples are isolated by conventional column chromatography or fractional crystallization. Unless otherwise indicated, all temperatures are in degrees Celsius.

Example 1

3-(N-Phthaloylglycyl)-2-(2-Hydroxyphenyl)-4-(R)-Thiazolidinecarboxylic Acid

A 4.5 g (0.02 mole) quantity of 2-(2-hydroxyphenyl)-4(R)-thiazolidinecarboxylic acid (prepared

according to H. Soloway *et al., JACS 70* 1667 (1948)) is suspended in 65 ml of methylene chloride in a 250 ml flask equipped with a stirring bar and calcium chloride drying tube, and treated with 4.55 g (0.045 mole) of triethyl amine. The reaction mixture, now a light-yellow solution, is chilled in an ice bath (0°) for 15 minutes, then treated with 4.47 g (0.02 mole) of phthaloylglycyl chloride. After 2 minutes of stirring at 0°C, the ice bath is removed and stirring continued at room temperature for 3 hours. The clear yellow solution is then made weakly acidic by careful addition of 2.5N HCl. The organic layer is washed twice with water, once with saturated salt solution, dried over magnesium sulfate and concentrated *in vacuo* to a yellow foam. Trituration of this foam with ether provides 4.15 g of a light yellow solid. Chromatography over silica gel (chloroform-methanol; 15:1) provides material with a good nmr spectrum in deuteromethanol: phthalamido aromatic doublet at 7.75 ppm; phenolic aromatic multiplet at 6.98 ppm; singlets at 6.52 and 3.4 ppm; and multiplets at 5.0 and 4.2 ppm.

Example 2

3-Glycyl-2-(2-Hydroxyphenyl)-4(R)-thiazolidinecarboxylic acid

A. A 500 mg (1.21 millimole) quantity of 3-(N-phthaloylglycyl)-2-(2-hydroxyphenyl)-4(R)-thiazolidinecarboxylic acid can be suspended in 5 ml of anhydrous ethyl alcohol in a 25 ml flask equipped with a stirring bar and reflux condenser with a calcium chloride drying tube. 1.21 ml of a 1M solution of hydrazine hydrate in absolute ethyl alcohol can be added, and the resulting mixture stirred and refluxed for 1 hour. During this time, all solid should go into solution, followed by the formation of a white precipitate. After cooling to room temperature, the reaction mixture can be evaporated *in vacuo,* and the residual solid stirred with 4 ml of 2N HCl at 50°C for 10 minutes, then allowed to slowly cool to room temperature over a 30 minute period. The mixture can then be filtered to remove the phthalylhydrazide and the filtrate concentrated *in vacuo* to a yellow foam which can be dissolved in 20 ml of water and placed on a column of Dowex 50W—X2($H^+$). Following a water wash, the column can be eluted with a total of 100 ml of water containing 2% pyridine. Lyophilizing the basic fraction can provide crude 3-glycyl-2-(2-hydroxyphenyl)-4(R)-thiazolidinecarboxylic acid.

B. Alternatively, a solution of 2.72 g (0.01 mole) of N-*t*-butoxycarbonylglycine-N-hydroxysuccinimide ester (prepared according to Anderson *et al., JACS 86* 1839 (1969)) in 20 ml of dioxane can be added to a solution of 3.38 g (0.015 mole) of 2-(2-hydroxyphenyl)-4-thiazolidinecarboxylic acid and 1.26 g (0.015 mole) of sodium bicarbonate dissolved in 16 ml of water at room temperature. After 1 hour, 10 ml more water can be added and the solution acidified to pH 2 with conc. HCl. The mixture can then be chilled, and the precipitated solid can be collected by filtration. The crude *t*-butyloxycarbonyl derivative which can then be obtained can then, after drying, be stirred in 25—30 ml of a 4N HCl-ethyl acetate solution at 0°C for 1 hour. Evaporation of the solvent and excess HCl can provide crude 3-glycyl-2-(2-hydroxyphenyl)-4(R)-thiazolidinecarboxylic acid as the hydrochloride salt. This material can then be purified and converted to the free base by eluting it from a column of Dowex-50W ($H^+$) with water containing 2% pyridine.

Example 3

3-(N-Benzyloxycarbonylglycyl)-2-(2-hydroxyphenyl)-4(R)-thiazolidinecarboxylic acid

*Method A.* A solution of N-benzyloxycarbonylglycine (0.50 g., 0.0024 mole) in 5 ml of HMPA (hexamethylphosphoramide) and 1.5 ml of acetonitrile at −5° was treated dropwise with thionyl chloride (0.30 g., 0.0024 mole) over 15 min. After an additional 10 min. at −5° the clear yellow solution was added dropwise over 20 min. to a solution of 2-(2-hydroxyphenyl-4(R)-thiazolidinecarboxylic acid (0.53 g., 0.0024 mole) in 2.4 ml of 1N NaOH and 4.8 ml of 1M $K_2CO_3$ at −5°. The clear solution was allowed to warm to room temperature over 3 hours, was added to 25 ml of $H_2O$, and was acidified to pH 2 with 7.2 ml of 1N HCl. The solution was extracted with 2 × 50 ml of ethylacetate which were washed with 25 ml of $H_2O$ and 25 ml of saturated NaCl solution. The combined organics were dried over 4 g. of $MgSO_4$, filtered and concentrated to an oil which was chromatographed on a 240 × 2.5 cm LH—20 column with 20 ml fractions of methanol. Concentration of fractions 56—66 gave 0.54 g. (54%) of a solid: nmr consistent with structure; mass spectrum (trimethylsilyl) m/e: 632 (M + three silyl), 488 (M + two silyl); tlc (Anal. tech. SGF) Rf = 0.92 (5-ethyl acetate, 1-acetic acid, 1-n-butanol, 1-water).

*Method B.* A solution of N-benzyloxycarbonylglycine (0.50 g., 0.0024 mole) in 5 ml of DMF at −5° was treated dropwise with thionylchloride (0.30 g., 0.0024 mole) over 5 min. After an additional 10 min. of stirring, the solution was added dropwise over 10 min. to a solution of 2-(2-hydroxyphenyl)-4(R)-thiazolidinecarboxylic acid (0.53 g., 0.0024 mole) in $CH_2Cl_2$ (5 ml) containing triethylamine (0.66 ml, 0.0048 mole) at −5°. After an additional 30 min. at −5° the solution was allowed to warm to room temperature overnight. Work up as before gave 0.12 g (12%) of solid which was identical with material from method A.

Example 4

3-(Chloroacetyl)-2-(2-hydroxyphenyl)-4(R)-thiazolidinecarboxylic acid

A portion of 2-(2-hydroxyphenyl)-4(R)-thiazolidinecarboxylic acid (0.53 g, 0.0024 mole) was dissolved in a mixture of 2.4 ml of 1M NaOH and 4.8 ml of 1M $K_2CO_3$. While stirring at −5°, chloroacetyl chloride (0.29 g, 0.0026 mole) was added dropwise over 15 min. After stirring at −5° for 1 hr. the solution was allowed to warm to room temperature overnight. The pH was reduced to 2 with 1M HCl and the solution was extracted with 2 × 25 ml of ethyl acetate. The organics were washed with 25 ml of $H_2O$ and 25 ml of saturated NaCl solution. They were combined and dried over 4 g of $MgSO_4$.

Example 5

3-Glycyl-2-(2-hydroxyphenyl)-4(R)-thiazolidinecarboxylic acid

*Method A;* A solution of 3-(N-benzyloxycarbonylglycyl)-2-(2-hydroxyphenyl)-4(R)-thiazolidinecarboxylic acid (0.63 g, 0.00151 mole) in 25 ml of 30% HBr in acetic acid was stirred at room temperature for 50 min. Excess HBr was blown off with a rapid stream of $N_2$ for 15 min. and the acetic acid was removed *in vacuo.* Purification over a 25 cc Dowex 50 (2X) column with 25 ml fractions of 3% pyridine in $H_2O$ gave, in fractions 3—6, 0.20 g (47%) of white solid; nmr consistent with structure; mass spectrum (FAB) m/e: 283 (M+H); tlc (Anal. tech. SGF) Rf = 0.70 (1-ethyl acetate, 1-acetic acid, 1-n-butanol, 1—$H_2$—O) and 0.30 (5-ethyl acetate, 1-acetic acid, 1-n-butanol, 1—$H_2O$).

*Method B:* A portion of crude 3-(chloroacetyl)-2-(2-hydroxyphenyl)-4(R)-thiazolidinecarboxylic acid (0.44 g, 0.00146 mole) was dissolved in 25 ml of concentrated $NH_4OH$ and stirred overnight at room temperature. After concentrating to dryness, the crude product was purified on a 25 cc Dowex 50 (2X) column, eluting with 25 ml fractions of 3% pyridine in $H_2O$. Concentration of fractions 3—6 gave 0.060 g (14%) of white solid which was identical with material from Method A.

Example 6

The keto acids and keto esters listed below in Table I can be reductively condensed with intermediates

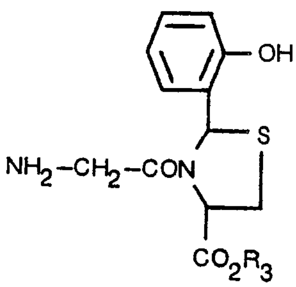 , 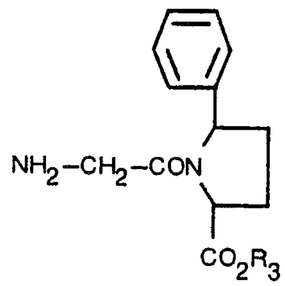 and 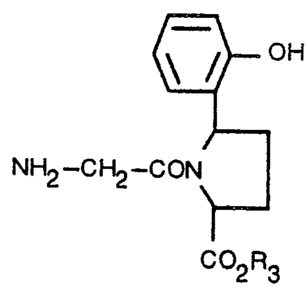

by the syntheses methods disclosed in this invention to yield, after removal of protecting groups, if any, the compounds of Formula I listed hereinbelow in Table II. In the foregoing intermediates, $R_3$ can be hydrogen or ethyl or benzyl groups, the latter ester groups being introduced on the thiaproline or proline materials under Fisher esterification conditions

TABLE I

Keto Acids and Keto Esters of the Formula:

$$R_1COCO_2R$$

(II)

| | | | |
|---|---|---|---|
| (a) | 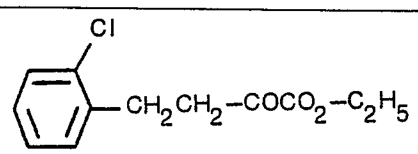 | (b) | 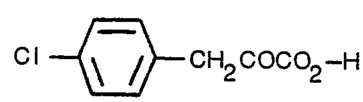 |
| (c) | 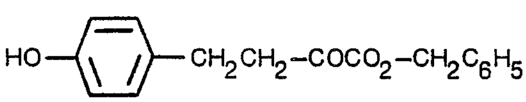 | (d) | * 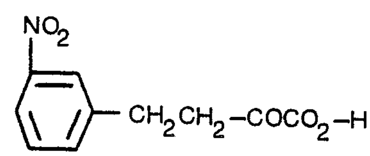 |
| (e) | 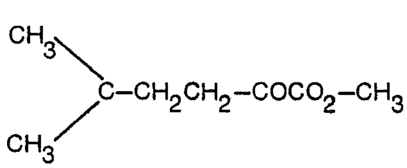 | (f) | 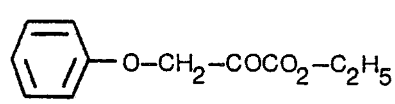 |

* Precursor to m-amino derivatives by $H_2$ /Pd.

TABLE II

Additional Products of Formula I:

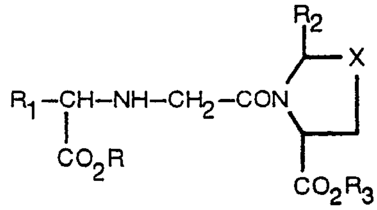

| | R | $R_1$ | $R_2$ | X | $R_3$ |
|---|---|---|---|---|---|
| (1) | $C_2H_5$ | 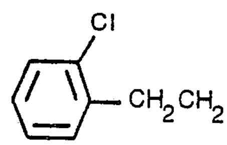 | 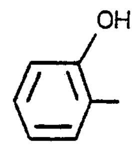 | S | H |
| (2) | H | 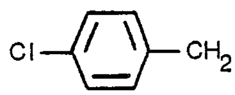 | 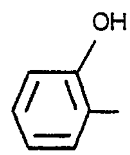 | S | $C_2H_5$ |
| (3) | $C_6H_5CH_2$ | 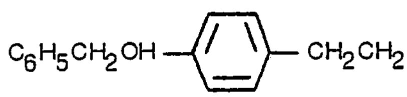 | 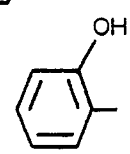 | S | H |
| (4) | H | 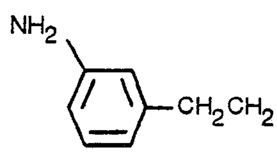 | 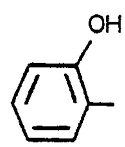 | S | H |
| (5) | $CH_3$ | 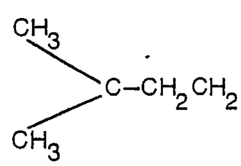 | 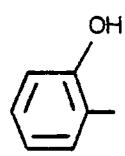 | S | 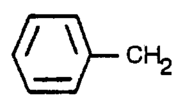 |
| (6) | $C_2H_5$ | 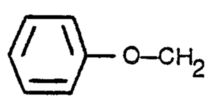 | 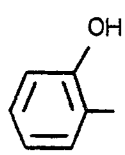 | S | H |

**Claims for the Contracting States: BE CH DE FR GB IT LI LU NL SE**

1. A compound of the formula:

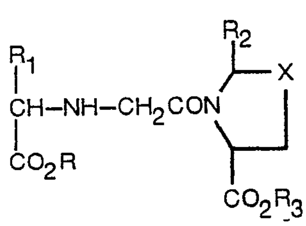

(I)

wherein:

R and $R_3$ are the same or different and are hydrogen, loweralkyl, aralkyl;

$R_1$ is alkyl of from one to ten carbon atoms which include, branched, cyclic and unsaturated alkyl groups; substituted alkyl of from one to six carbon atoms wherein the substituent is amino, arylthio, aryloxy, hydroxy, arylamino, and acylamino; or, aralkyl and heteroaralkyl substituted by halo, loweralkyl, hydroxy, aminoloweralkyl, alkoxy and amino groups and wherein the alkyl group contains from one to six carbon atoms;

$R_2$ is an aryl, substituted aryl, heteroaryl or substituted heteroaryl group selected from the group consisting of phenyl, nephthyl, or biphenyl wherein the substituent is hydroxyl or lower alkyloxy;

X is $CH_2$ or S; and, the pharmaceutically acceptable salts thereof.

2. A pharmaceutical composition useful in the treatment of hypertension which comprises a pharmaceutically acceptable carrier and a pharmaceutically effective amount of a compound of claim 1.

3. The composition of Claim 2 which includes another antihypertensive and/or diuretic compound selected from the group consisting of hydrochlorothiazide, methyldopa, timolol, indacrinone, the pivaloyoxyethyl ester of methyldopa, 4-{3-{[2-(1-hydroxycyclohexyl)ethyl]-4-oxo-2-thiazolidinyl}propyl}benzoic acid as well as combinations and mixtures thereof.

4. A process for preparing a compound of claim 1 which process comprises reacting a ketone of the formula:

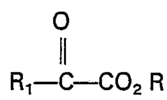

wherein R and $R_1$ are as defined above, in the presence of sodium cyanoborohydride in an aqueous solution at neutral pH or in a suitable organic solvent with a dipeptide of the formula:

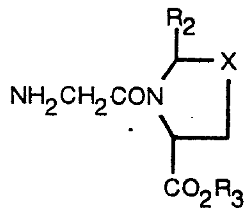

wherein $R_2$, $R_3$ and X are as defined above, to afford the desired product after removal of protective groups, if any, if desired, obtaining the biologically more active diastereomer thereof by chromatography or fractional crystallization and, if further desired, preparing a pharmaceutically acceptable salt thereof by conventional means.

**Claim for the Contracting State: AT**

1. A process for preparing a compound of the formula:

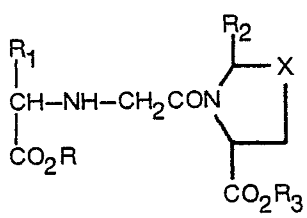

(I)

R and $R_3$ are the same or different and are hydrogen, loweralkyl, aralkyl;

$R_1$ is alkyl of from one to ten carbon atoms which include, branched, cyclic and unsaturated alkyl of from one to six carbon atoms wherein the substituent is amino, arylthio, aryloxy, hydroxy, arylamino, and acylamino; aralkyl and heteroaralkyl substituted by halo, loweralkyl, hydroxy, aminoloweralkyl, alkoxy and amino groups and wherein the alkyl group contains from one to six carbon atoms;

$R_2$ is an aryl, substituted aryl, heteroaryl or substituted heteroaryl group selected from,the group consisting of phenyl, naphthyl, or biphenyl wherein the substituent is hydroxyl or lower alkyloxy;

X is $CH_2$ or S; which process comprises reacting a ketone of the formula:

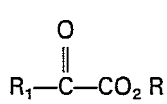

wherein R and $R_1$ are as defined above, in the presence of sodium cyanoborohydride in an aqueous solution at neutral pH or in a suitable organic solvent with a dipeptide of the formula:

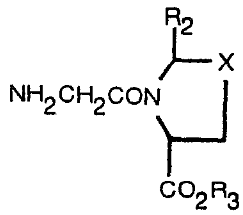

wherein $R_2$, $R_3$ and X are as defined above, to afford the desired product after removal of protective groups, if any, and, if desired, obtaining the biologically more active diastereomer thereof by chromatography or fractional crystallization and, if further desired, preparing a pharmaceutically acceptable salt thereof by conventional means.

**Patentansprüche für die Vertragsstaaten: BE CH DE FR IT LI LU NL SE**

1. Eine Verbindung der Formel:

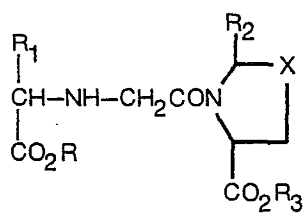

(I)

worin:

R und $R_3$ gleich oder verschieden sind und Wasserstoff, Niedrigalkyl oder Aralkyl bedeuten;

$R_1$ Alkyl mit einem bis zehn Kohlenstoffatomen, darunter verzweigte, cyclische und ungesättigte Alkylgruppen; substituiertes Alkyl mit einem bis sechs Kohlenstoffatomen, wobei der Substituent Amino, Arylthio, Aryloxy, Hydroxy, Arylamino und Acylamino, ist; oder Aralkyl und Heteroaralkyl, das durch Halogen, Niedrigalkyl, Hydroxy, Aminoniedrigalkyl, Alkoxy und Aminogruppen substituiert ist, und wobei die Alkylgruppe ein bis sechs Kohlenstoffatome enthält, ist; eine Aryl-, substituierte Aryl-, Heteroaryl- oder substituierte Heteroarylgruppe ist, die aus der Gruppe ausgewählt ist, welche aus Phenyl, Naphthyl oder Biphenyl besteht, wobei der Substituent Hydroxyl oder Niedrigalkyloxy ist;

X $CH_2$ oder S ist; und die pharmazeutisch annehmbaren Salze davon.

2. Eine pharmazeutische Zusammensetzung, die zur Behandlung von Hypertension nützlich ist, und welche einen pharmazeutisch annehmbaren Träger und eine pharmazeutisch wirksame Menge einer Verbindung von Anspruch 1 enthält.

3. Die Zusammensetzung des Anspruchs 2, welche eine weitere antihypertensive und/oder diuretische Verbindung enthält, die aus der Gruppe ausgewählt ist, welche aus Hydrochlorothiazid, Methyldopa, Timolol, Indacrinon, dem Pivaloyloxyethylester von Methyldopa 4-{3-{[2-(1-Hydroxycyclohexylethyl]-4-oxo-2-thiazolidinyl}propyl}benzoesäure, sowie Kombinationen und Mischungen davon, besteht.

4. Ein Verfahren zur Herstellung einer Verbindung von Anspruch 1, welches Verfahren das Umsetzen eines Ketons der Formel:

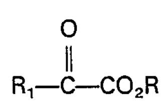

,

worin R und $R_1$ wie oben definiert sind, in Gegenwart von Natriumcyanoborhydrid in einer wässerigen Lösung bei neutralem pH oder in einem geeigneten organischen Lösungsmittel, mit einem Dipeptid der Formel:

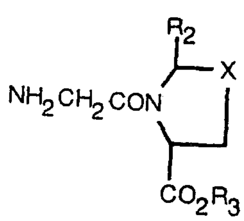

,

worin $R_2$, $R_3$ und X wie oben definiert sind, um, gegebenenfalls nach Entfernung von Schutzgruppen, das gewünschte Produkt zu erhalten, und gewünschtenfalls das Gewinnen des biologisch aktiveren Diastereomers davon durch Chromatographie oder fraktionierte Kristallisation, und, falls weiterhin erwünscht, das Herstellen eines pharmazeutisch annehmbaren Salzes davon durch übliche Mittel umfaßt.

**Patentanspruch für den Vertragsstaat: AT**

1. Ein Verfahren zur Herstellung einer Verbindung der Formel:

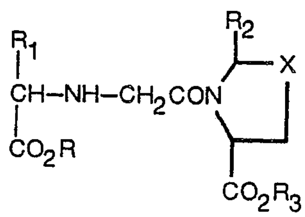

(I)

worin:

R und $R_3$ gleich oder verschieden sind und Wasserstoff, Niedrigalkyl oder Aralkyl bedeuten;

$R_1$ Alkyl mit einem bis zehn Kohlenstoffatomen, darunter verzweigte, cyclische und ungesättigte Alkylgruppen; substituiertes Alkyl mit einem bis sechs Kohlenstoffatomen, wobei der Substituent Amino, Arylthio, Aryloxy, Hydroxy, Arylamino und Acylamino, ist; oder Aralkyl und Heteroaralkyl, das durch Halogen, Niedrigalkyl, Hydroxy, Aminoniedrigalkyl, Alkoxy und Aminogruppen substituiert ist, und wobei die Alkylgruppe ein bis sechs Kohlenstoffatome enthält, ist;

$R_2$ eine Aryl-, substituierte Aryl-, Heteroaryl- oder substituierte Heteroarylgruppe ist, die aus der Gruppe ausgewählt ist, welche aus Phenyl, Naphthyl oder Biphenyl besteht, wobei der Substituent Hydroxyl oder Niedrigalkyloxy ist;

X $CH_2$ oder S ist; welches Verfahren das Umsetzen eines Ketons der Formel:

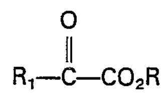 ,

worin R und $R_1$ wie oben definiert sind, in Gegenwart von Natriumcyanoborhydrid in einer wässerigen Lösung bei neutralem pH oder in einem geeigneten organischen Lösungsmittel, mit einem Dipeptid der Formel:

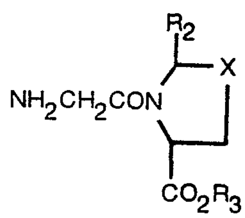

worin $R_2$, $R_3$ und X wie oben definiert sind, um, gegebenenfalls nach Entfernung von Schutzgruppen, das gewünschte Produkt zu erhalten, und gewünschtenfalls das Gewinnen des biologisch aktiveren Diastereomers davon durch Chromatographie oder fraktionierte Kristallisation, und, falls weiterhin erwünscht, das Herstellen eines pharmazeutisch annehmbaren Salzes davon durch übliche Mittel umfaßt.

**Revendications pour les Etats contractants: BE CH DE FR GB IT LI LU NL SE**

1. Composé de formule

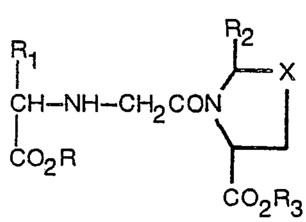

(I)

où

R et R3 sont semblables ou différents et représentent un hydrogène, alcoyle inférieur, aralcoyle;

R1 est un alcoyle ayant de un à dix atomes de carbone comprenant les groupes alcoyle à chaîne rampfiée, cycliques et non saturés; un alcoyle substitué ayant de un à six atomes de carbone où le

substituant est un amino, arylthio, aryloxy, hydroxy, arylamino, et acylamino; un aralcoyle et un hétéro-alcoyle substitués par des groupes halo, alcoyle inférieur, hydroxy, aminoalcoyle inférieur, alcoxy et amino et où le groupe alcoyle contient de un à six atomes de carbone;

R2 est un groupe aryle, aryle substitué, hétéroaryle ou hétéroaryle substitué choisi dans le groupe constitué par phényle, naphtyle ou biphényle où le substituant est un hydroxyle ou un alcoyloxy inférieur;

X représente $CH_2$ ou S; et ses sels pharmaceutiquement acceptables.

2. Composition pharmaceutique utile dans le traitement de l'hypertension qui comprend un support pharmaceutiquement acceptable et une quantité pharmaceutiquement efficace d'un composé de la revendication 1.

3. Composition de la revendication 1 qui comprend un autre composé antihypertenseur et/ou diurétique choisi dans le groupe constitué par hydrochlorothiazide, méthyldopa, timolol, indacrinone, le pivaloyloxyéthyl-ester de méthyldopa, l'acide 4-{3-{[2(1-hydroxycyclohéxyl)éthyl]-4-oxo-2-thiazolidinyl}propyl} benzoïque ainsi que leurs combinaisons et mélanges.

4. Procédé de préparation d'un composé de la revendication 1, lequel procédé comprend la réaction d'une cétone de formule

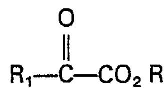

où R et R1 sont tels que définis ci-dessus, en présence de cyanoborohydrure de sodium dans une solution aqueuse à pH neutre ou dans un solvant organique approprié avec un dipeptide de formule:

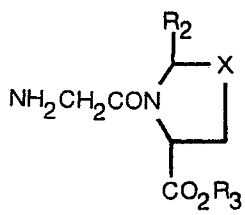

où R2, R3 et X sont tels que définis ci-dessus, pour donner le produit désiré après enlèvement des groupes protecteurs, s'il y en a, l'obtention de son diastéréomère biologiquement plus actif par chromatographie ou cristallisation fractionnée et, si on le désire encore, la préparation d'un de ses sels pharmaceutiquement acceptables par des moyens classiques.

**Revendication pour l'Etat contractant: AT**

Procédé de préparation d'un composé de formule:

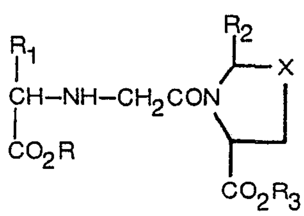

(I)

où

R et R3 sont semblables ou différents et représent un hydrogène, alcoyle inférieur, aralcoyle;

R1 est un alcoyle ayant de un à dix atomes de carbone comprenant les groupes alcoyle à chaîne rampfiée, cycliques et non saturés; un alcoyle substitué ayant de un à six atomes de carbone où le substituant est un amino, arylthio, aryloxy, hydroxy, arylamino, et acylamino; un aralcoyle et un hétéro-alcoyle substitués par des groupes halo, alcoyle inférieur, hydroxy, aminoalcoyle inférieur, alcoxy et amino et où le groupe alcoyle contient de un à six atomes de carbone;

R2 est un groupe aryle, aryle substitué, hétéroaryle ou hétéroaryle substitué choisi dans le groupe constitué par phényle, naphtyle ou biphényle où le substituant est un hydroxyle ou un alcoyloxy inférieur;

X représente $CH_2$ ou S; procédé comprenant la réaction d'une cétone de formule:

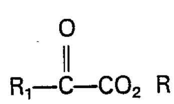

où R et R1 sont tels que définis ci-dessus, en présence de cyanoborohydrure de sodium dans une solution aqueuse à pH neutre ou dans un solvant organique approprié avec un dipeptide de formule:

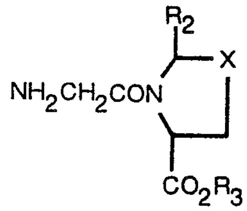

où R2, R3 et X sont tels que définis ci-dessus, pour donner le produit désiré après enlèvement des groupes protecteurs, s'il y en a, l'obtention de son diastéréomère biologiquement plus actif par chromatographie ou cristallisation fractionnée et, si on le désire encore, la préparation d'un de ses sels pharmaceutiquement acceptables par des moyens classiques.